# EUROPEAN PATENT APPLICATION

(11) **EP 4 471 792 A1**
(43) Date of publication of application: **04.12.2024**
(21) Application number: 23176286.5
(22) Date of filing: 31.05.2023
(51) Int. Cl.: G16H 40/20, G16H 40/63, G16H 40/67

(54) **CONTROLLING THE REOCCURANCE OF AN ALARM**

(71) Applicant: Koninklijke Philips N.V., 5656 AG Eindhoven (NL)
(72) Inventor: VAN LIESHOUT, Ron Martinus Laurentius, 5656 AG Eindhoven (NL); HUIJBREGTS, Laurentia Johanna, 5656 AG Eindhoven (NL); DANISMAN-TASAR, Mine, 5656 AG Eindhoven (NL)
(74) Representative: Philips Intellectual Property & Standards

(57) **Abstract**

A mechanism for controlling the length of time for which an alarm, for a subject undergoing physiological monitoring, is silenced. Information responsive to any change in the condition of the subject or of any clinicians attending to the subject is used to control the minimum length for the silence period (during which time the alarm is silenced).

## Description

### FIELD OF THE INVENTION

The present invention relates to the field of patient monitoring.

### BACKGROUND OF THE INVENTION

When monitoring an entity, there is a desire to trigger alarms to indicate deviance from a normal or expected operation of that entity. This can be used to identify when that entity needs attention or is operating in an undesired state.

Such methods of generating an alarm are especially important in a clinical setting to enable clinicians to respond to a deterioration in a subject's or patient's condition. Typically, a subject's physiological parameters (e.g., heart-rate, SpO2 percentage, respiratory rate and so on) undergo continuous monitoring and alarms are generated based on the monitored physiological parameters.

Traditionally, an alarm can be triggered when a value of a particular physiological parameter breaches (e.g., goes above or below) a particular threshold. For example, for an adult male, an alarm may be triggered when their heartrate falls below 60 beats per minute (bpm) or rises above 200 bpm.

Alarms can be broadly divided into two categories: critical and non-critical. A critical alarm indicates that there is an urgent need to attend to the subject and a non-critical alarm indicates there is no urgent need, e.g., provides information only.

There has been a recent trend towards the use of predictive algorithms that process values of different physiological parameters in order to produce an alarm parameter having a single value or indicator. Examples include a hemodynamic stability index (HSI), an output of a sepsis prediction model, the ROX index and so on. It is possible to trigger an alarm when one of these values/indexes indicates there is a need or desire for clinical review. This may occur when the value/index passes a particular threshold.

Thus, it is possible to more broadly define an alarm parameter, the value of which controls the triggering of an alarm. In particular, an alarm may be generated if the value(s) of one or more alarm parameters meets a certain criterion/criteria, e.g., exceeds a threshold. The alarm parameter may be a physiological parameter or a parameter derived from one or more physiological parameters.

It has been recognized that providing an excess of alarms to a clinician can lead to alarm fatigue. Alarm fatigue leads to desensitization of clinicians (or other observers) to alarms, causing the clinicians to repeatedly silence an alarm, ignore an alarm or disbelieve the significance of an alarm.

There is an ongoing desire to reduce alarm fatigue in clinicians.

### SUMMARY OF THE INVENTION

The invention is defined by the claims.

According to examples in accordance with an aspect of the invention, there is provided a computer-implemented method of controlling the reoccurrence of an alarm for a subject undergoing physiological patient monitoring.

The computer-implemented method comprises, responsive to the occurrence of an alarm: obtaining first information that changes responsive to any changes in a condition of the subject and/or any changes in a condition of one or more clinicians responsible for the subject; processing the first information to determine a minimum length for a silence period, being a period for which the alarm is to remain silent after an indication to silence the alarm is received; and responsive to receiving the indication to silence the alarm, initiating the silence period and preventing the alarm from reoccurring at least until an elapsed length of the silence period breaches the minimum length for the silence period.

The present disclosure provides a technique for dynamic adaptation of the silence period between occurrence and reoccurrence of an alarm. This allows for the length of the silence period to adapt to the condition of the subject and/or an attention/availability of the clinician (amongst other properties of a subject/clinician). Embodiments recognize that a wide variety of factors are able to influence the period between occurrences of a same alarm to advantage.

The alarm is preferably a user-perceptible alarm that is generated at a user interface, such as a visual alarm, an audible alarm and/or a haptic alarm. Approaches for generating user-perceptible alarms are well known to the skilled person.

The alarm may be a critical alarm or a non-critical alarm (i.e., a notification). An alarm is therefore any perceptible output that is generated responsive to a (monitored, calculated or predicted) parameter of the subject breaching a certain threshold and/or meeting one or more other predetermined criteria (e.g., falling in a particular range or the like).

The computer-implemented method may further comprise, during the silence period, iteratively: obtaining updated first information; and processing the updated first information to redetermine the minimum length for the silence period. This approach advantageously performs iterative updating of the minimum length of the silence period in order to dynamically react and respond to any ongoing changes with the subject and/or the clinician(s) responsible for the subject. This reduces a risk that unexpected changes in the condition of the subject will be missed due to an alarm silence.

In some examples, the first information comprises clinician information that changes responsive to an availability of the one or more clinicians to attend to the subject; and processing the first information comprises processing at least the clinician information.

In some examples, the clinician information comprises schedule information that indicates a schedule of the one or more clinicians.

In some examples, the clinician information comprises attention information that indicates a level of attention paid by the clinician to the subject undergoing physiological patient monitoring.

In some examples, the clinician information comprises location information that indicates a proximity of the one or more clinicians to the subject undergoing physiological patient monitoring.

In some examples, the first information comprises subject condition information that changes responsive to any changes in the condition of the subject; and processing the first information comprises processing at least the subject information.

In some examples, the alarm is responsive to a value of at least one alarm parameter derived from one or more monitored physiological parameters; and the subject condition information changes responsive to any changes in the value of each alarm parameter.

In some examples, the first information comprises subject intervention information that changes responsive to the occurrence of any clinical interventions performed on the subject; and processing the first information comprises processing at least the subject intervention information.

The method may further comprise, responsive to receiving an indication to silence the alarm, controlling a user interface to provide a user-perceptible output responsive to minimum length for the silence period.

The method may further comprise, responsive to the occurrence of an alarm: processing the first information to determine a maximum length for the silence period; and responsive to receiving an indication to silence the alarm, triggering the reoccurrence of the alarm when the maximum length for the silence period has elapsed.

The method may further comprise, responsive to receiving an indication to silence the alarm after the occurrence of the alarm, triggering the reoccurrence of the alarm responsive to: the elapsed length of the silence period falling between the minimum length and the maximum length of the silence period; and receiving a trigger signal for triggering the reoccurrence of the alarm.

In some examples, the trigger signal is an indication that a clinician is interacting or observing the subject. In another example, the trigger signal is an indication that one or more alarm parameter(s) meet one or more predetermined criteria, e.g., breaching a threshold.

There is also provided a computer program product comprising computer program code means which, when executed on a computing device having a processing system, cause the processing system to perform all of the steps of the method according to any herein disclosed method.

There is also provided a processing system for controlling the reoccurrence of an alarm for a subject undergoing physiological patient monitoring, the processing system being configured to, responsive to the occurrence of an alarm: obtain first information that changes responsive to a condition of the subject and/or one or more clinicians responsible for the subject; process the first information to determine a minimum length for a silence period, being a period for which the alarm is to remain silent after an indication to silence the alarm is received; and responsive to receiving the indication to silence the alarm, initiate the silence period and prevent the alarm from reoccurring at least until an elapsed length of the silence period breaches the minimum length for the silence period.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a subject-monitoring system;
Fig. 2 illustrates a method according to an embodiment;
Fig. 3 illustrates another method according to an embodiment.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for controlling the length of time for which an alarm, for a subject undergoing physiological monitoring, is silenced. Information responsive to any change in the condition of the subject or of any clinicians attending to the subject is used to control the minimum length for the silence period (during which time the alarm is silenced).

Approaches thereby provide an approach that is able to dynamically modify the length of the silence period based on any changes in circumstance that can affect an urgency of an alarm or an availability of a clinician to attend to an alarm. This can help ensure that the alarm reoccurs when it is necessary (from a clinical perspective) or when it is best for a clinician to review the subject, without reoccurring too early.

In the context of the present disclosure, an alarm is any alert, notification, prompt or message that is generated responsive to one or more values of one or more alarm parameters (derived from monitored physiological measurements) meeting one or more predetermined criteria. Typically, an alarm is generated responsive to at least a value of an alarm parameter breaching some predetermined threshold or a combination of different values breaching different predetermined thresholds.

An alarm parameter may be a monitored physiological measurement (e.g., a heart rate or respiratory rate) or a parameter derived from processing physiological measurements, such as a hemodynamic stability index (HSI). One example approach for determining an HSI is disclosed by Rahman, Asif, et al. "Early prediction of hemodynamic interventions in the intensive care unit using machine learning." Critical Care 35.1 (2021): 2-9.

The term alarm is therefore not to be considered as merely including critical alarms (that indicate a clinically dangerous deterioration or condition of subject), but rather may further include non-critical alarms (e.g., warning alarms, notifications, prompts for action, advisory messages and so on).

An alarm may be associated with a user perceptible output, e.g., at a user interface. Thus, when an alarm occurs, a user perceptible output for the alarm may be provided at the user interface. For instance, a siren may sound, a light may flash, a prompt or message may be generated or displayed and so on. A wide variety of different concepts and approaches for providing a user-perceptible output of an alarm are known to the appropriately skilled person in the art.

In the context of the present disclosure, a clinician is any health care professional with responsibility for the subject undergoing physiological monitoring, and may include a physician/doctor, a nurse, a nurse practitioner, a caregiver, a carer, a physician assistant and so on.

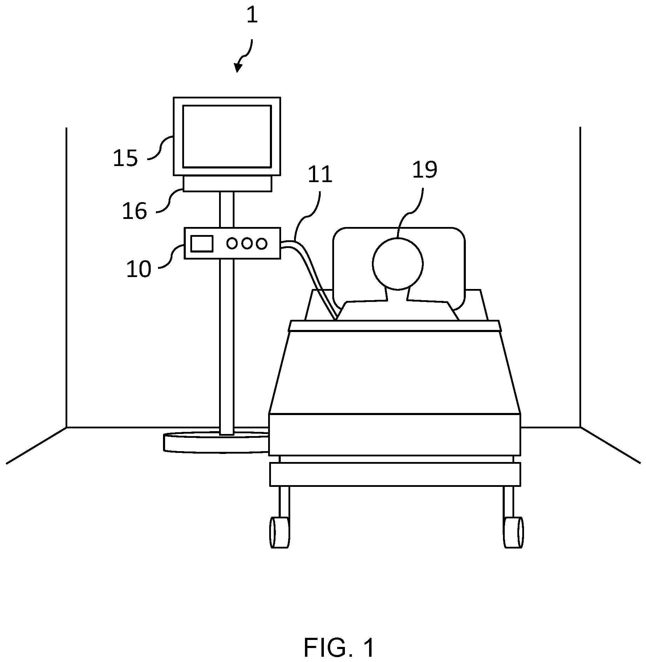

Fig. 1 illustrates a subject-monitoring system 1 in which embodiments of the invention can be employed. The subject-monitoring system 1 comprises a subject-monitoring device 10 and an output generator 15.

The subject-monitoring device 10 is adapted to monitor one or more physiological parameters of a subject 19. This may be performed, for example, using one or more physiological parameter monitors (not shown) coupled to the subject-monitoring device via cables 11 or wirelessly. Examples of suitable physiological parameter monitors include a pulse oximeter (for measuring SpO₂ level and/or heartrate), a heart rate monitor (for measuring heart rate), a movement sensor (for measuring respiratory rate), a blood pressure monitor (for measuring blood pressure) and so on.

The subject-monitoring device 10 is adapted to obtain the physiological parameters and generate alarms. An alarm may indicate, for example, the occurrence of a clinically undesirable level of an alarm parameter. As previously mentioned, an alarm parameter may be derived from (e.g., equal to or produced by processing) at least one physiological parameter.

One alarm may, for example, be generated responsive to a value of a physiological parameter breaching a predetermined threshold (e.g. rising above or falling below a predetermined threshold, depending upon the type of the physiological parameter). Another alarm may indicate, for example, an error or warning of the subject-monitoring device (or physiological parameter monitor), such as a low battery level or availability of an update.

Examples of alarm include alarms indicating: low heart rate; high heart rate; arrhythmia; asystole; low breathing rate; high breathing rate; apnea; low SpO2; high SpO2; low temperature; high temperature; low systolic blood pressure; high systolic blood pressure; low diastolic blood pressure; high diastolic blood pressure; low mean blood pressure; high mean blood pressure; low perfusion; poor pallor (subject color); too little or too much subject movement; out of bed detection; low/high cardiac output; low/high stroke volume; low/high blood flow; low HSI; high septic risk and so on. Further examples of alarms (equipment-based) include, for a ventilator: high peak inspiratory pressure; disconnection of the ventilator circuit; malfunctioning/occluded endotracheal tube; and low CO₂ level. Yet further examples include, for an infusion pump: low flow or presence of air in the line. Further examples for subject-monitoring devices may include a "lead-disconnected" alarm or an "insufficient contact with subject" alarm. Various other alarms would be apparent to the skilled person, and could be integrated into the inventive concept.

Of course, the subject-monitoring device may comprise more than one subject-monitoring device, each adapted to monitor one or more physiological parameters of the subject.

The output generator 15 is adapted to obtain the alarms generated by the subject-monitoring device 10. The output generator 15 may communicate with the subject-monitoring device wirelessly or via one or more wires. Alarms may be communicated using respective alarm signals from the subject-monitoring device 10 to the output generator.

The output generator 15 may be configured to generate or produce one or more user-perceptible (i.e., clinician-perceptible) outputs responsive to the alarm. Generating a user-perceptible output may comprise generating an output signal that causes an output device (such as screen, speaker or the like) to provide a visual, audio and/or haptic output to a clinician.

The user-perceptible output can comprise any visual, audio or haptic output or alarm, which may be output by any visual, audio and/or haptic system, such as a mobile phone, tablet, monitoring device and so on. By way of example, a visual output may provide textual information on the alarm(s). An audio output may draw attention to the occurrence of the alarm(s), and may optionally provide further information (e.g., in the form of text-to-speech). A haptic output may alert a clinician to the occurrence of an alarm, e.g. with a number of vibrations indicating a number of alarms being notified at a particular point in time.

The output generator 15 is also configured to receive any indication(s) to silence any alarm(s). The indication may, for instance, be provided via an input interface of the subject-monitoring device 10 or the output generator 15. As another example, the indication may be performed by a separate processing system (not shown), e.g., monitoring the environment surrounding the subject.

Responsive to a received indication, the output generator 15 silences the corresponding alarm, e.g., stops providing at least one clinical-perceptible output for that alarm. This may, for instance, comprise one or more of: stopping an audio output of the alarm (if provided), minimizing a visual output of an alarm; ending a vibration of a haptic output and so on.

The present disclosure provides a technique for determining the minimum length of a silence period for which a silenced alarm is to remain silent. Further embodiments may define a maximum length of this silence period. Embodiments can be employed, for instance, in the subject-monitoring system illustrated by Fig. 1.

By way of example, the output generator 15 may comprise a processing system 16 configured to control the reoccurrence of an/any alarm that has been silenced. In particular, the processing system 16 may be able to control at least the minimum length of the silence period, after which minimum length has elapsed the alarm is permitted to be provided again (e.g., if the conditions for triggering the alarm are reached).

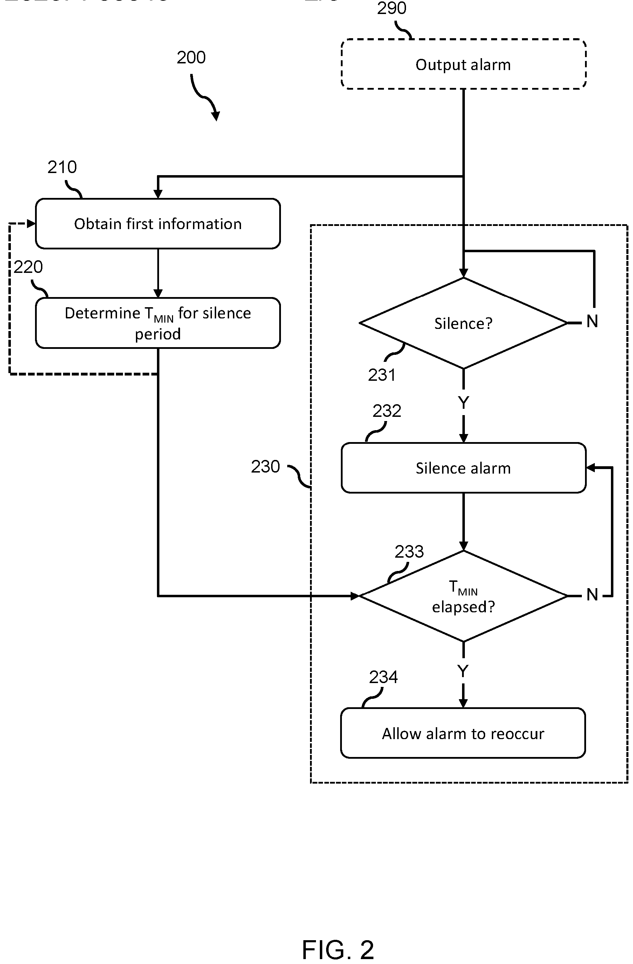

Fig. 2 is a flowchart illustrating a computer-implemented method according to an embodiment. The method 200 is controlling the reoccurrence of an alarm for a subject undergoing physiological patient monitoring. Thus, the method 200 may be carried out by a processing system 16 configured to control the operation of the output generator 15 previously described.

The steps of the computer-implemented method 200 are performed responsive to the occurrence of an alarm. Thus, the method 200 may be performed after an alarm occurs or is otherwise made active (e.g., in a step 290).

The method 200 comprises a step 210 of obtaining first information. The first information is information that changes responsive to any changes in a condition of the subject and/or any changes in a condition of one or more clinicians responsible for the subject.

The method 200 also comprises a step 220 of processing the first information to determine a minimum length T_{MIN} for a silence period. A minimum length is a minimum time period for which the alarm is to remain silent after an indication to silence the alarm is received. Thus, a silence period is a period of time for which the alarm is to remain silent.

Step 220 may, for instance, comprise modifying or changing a default minimum length for the silence period responsive to the first information. In particular, various optional pieces of information that may be contained in the first information may increase or decrease the value set for the minimum length T_{MIN} for the silence period - initially starting at the default length.

It will be appreciated that, when an alarm is silent, one or more user-perceptible outputs for the alarm are prevented from being generated or provided.

For instance, an audible alarm (e.g., a siren) may be stopped or silenced or a visual alarm (e.g., a flashing lamp or a pop-up notification) may be ended, stopped or closed. Various approaches for silencing an alarm are well known to the skilled person, and may depend upon the exact nature of the alarm and/or its associated user perceptible output(s).

The method 200 also comprises a step 230 of, responsive to receiving the indication to silence the alarm, initiating the silence period and preventing the alarm from reoccurring at least until an elapsed length of the silence period breaches the minimum length for the silence period.

Step 230 may therefore comprise a sub-step 231 of determining whether or not an indication to silence alarm has been received.

Various approaches for receiving an indication to silence an alarm are known and/or could be used in embodiments. As one example, an indication may be received in the form of a user input. As another example, an indication may be generated responsive to a determination that a clinician is not available to respond to the alarm (e.g., as they are attending another subject or are not in the room).

Responsive to a positive determination in sub-step 231, the step 230 may move to a sub-step 232 of silencing the alarm. Thus, sub-step 232 comprises initiating a silence period during which time the alarm is silent. Approaches for silencing an alarm have been previously described and are well known to the skilled person.

In some examples, sub-step 232 further comprises providing a user-perceptible output of the minimum time period and/or an identification that the alarm has been silenced. This provides a clinician with useful information (e.g., defining how long since an alarm was silenced) that can influence their clinical decision making. The user perceptible-output may be provided at the same output interface that provides a user-perceptible output of the (original) alarm.

Responsive to a negative determination in sub-step 231, the alarm is continued or not silenced. For instance, as illustrated, the step 230 may idle at sub-step 231.

After performing sub-step 232, the step 230 may perform a sub-step 233 of determining whether or not the minimum length T_{MIN} for the silence period has elapsed. Approaches for monitoring whether a length of time has elapsed are well known to the skilled person, and typically comprise determining a difference between a timestamp representing a start of the silence period and a timestamp representing a current point in time. Other approaches are known to the skilled person.

Responsive to a positive determination in sub-step 233, the step 230 may move to a sub-step 234 of permitting the alarm to reoccur. It is not essential that the alarm be immediately unsilenced at this stage (although this is, of course, possible) - but rather than the alarm is permitted to reoccur or become non-silent. By way of example, if an alarm condition is reached after the minimum length for the silence period has elapsed (e.g., a value of the alarm parameter breaches a predetermined threshold), then the alarm may again be generated or output.

In this way, the minimum length of the period of time for which the alarm is silent (after received an indication to silence) is responsive to changes in a subject condition and/or changes in a condition of one or more clinicians (e.g., caregivers) for the monitored subject. This advantageously allows for bespoke and/or dynamic updating of the minimum length of time (e.g., rather than a mere default silence period).

The proposed approach thereby defines the minimum length T_{MIN} based on changes that could affect the urgency of an alarm and/or an availability of a clinician to respond to an alarm. This can ensure timely action by a clinician to an alarm if its urgency is increased or they are able to respond to the alarm.

Of course, steps 210 and 220 can be iteratively repeated to repeatedly update or adjust the minimum length T_{MIN} used in step 230. This allows ongoing changes to the subject/clinician(s) during the silence period to be advantageously taken into account. In this way, the minimum length T_{MIN} can be dynamically based on (ongoing) changes that could affect the urgency of the alarm.

Thus, the method 200 may comprise, during the silence period, iteratively: obtaining updated first information; and processing the updated first information to redetermine the minimum length for the silence period. This iterative loop is illustrated with dashed lines.

The method 200 may be performed for each alarm and/or each reoccurrence of an alarm for the subject. Thus, the method 200 could be iteratively performed if an alarm repeatedly reoccurs.

To perform step 220, various parameters or pieces of information can be taken into account. A general principle around such pieces of information is that are preferably an indication that there is a change in the likelihood that the clinician can (e.g., is available to) and/or should (e.g., to avoid/mitigate a clinically dangerous state) perform one or more actions or interventions based on the alarm. From a high-level perspective, preferable (but not necessarily essential) pieces of information indicate: the availability of the clinician; any alteration of the patient state; and the availability of new information about the condition of the subject.

Thus, in some examples, the first information may contain clinician information that changes responsive to an availability of the one or more clinicians to attend to the subject. Processing the first information, in step 220, may comprises processing at least the clinician information.

The clinician information may, for instance, comprise schedule information or workshift information that indicates a schedule of the one or more clinicians. This facilitates control over the minimum length of the silence period responsive to the physical availability of the clinician.

As an example, it may be desirable for an alarm to reoccur for a same clinician (as was scheduled for the original alarm), such that the minimum length should be no greater than a time until the end of a shift for that clinician.

As another example, such schedule information can be used to derive information around the workload, which can be used to control the minimum time period.

As another example, the clinician information may comprise attention information that indicates a level of attention paid by the clinician to the subject undergoing physiological patient monitoring. A greater level of attention may reduce the minimum time period, as the clinician will be able to react to alarms that are output for the subject.

Attention information that indicates an attention of a clinician may include, for instance, an indication of whether or not: the clinician is in the same room as the subject; the clinician is looking at a health record of the subject; the clinician is looking towards the subject; the clinician is interacting with a monitoring device for the subject; the clinician is speaking to the subject and so on.

Another example of attention information is information on any other higher priority alarms for the clinician. When a high priority alarm is going off, the clinician has to first focus on this, such that they would not respond to the reoccurrence of a (less urgent) alarm. Accordingly, if there is at least further, higher priority alarm active, then the minimum length for the silence period may be increased.

As another example, the clinician information may comprise location information that indicates a proximity of the one or more clinicians to the subject undergoing physiological patient monitoring. The closer the clinician to the subject, the more likely that the clinician will be able to react or respond to an alarm. Accordingly, the minimum length for the silence period may be reduced responsive to clinician being proximate (e.g., a distance being below a predetermined distance threshold) to the subject.

In some examples, the first information comprises subject condition information that changes responsive to any changes in the condition of the subject. Processing the first information, in step 220, may comprise processing at least the subject information.

In some examples, the alarm is responsive to a value of at least one alarm parameter derived from one or more monitored physiological parameters. The subject condition information preferably changes responsive to any changes in the value of each alarm parameter. An alarm parameter is parameter that controls or defines when the alarm is generated. Thus, the alarm (produced in step 290) may be responsive to at least the alarm parameter.

Thus, in some examples, the subject condition information may comprise information that changes responsive to any change in a value (of an alarm parameter) that initially triggered the alarm to be silenced. For instance, the subject condition information may contain an indicator on whether or not the value(s) for the alarm no longer meet the criterion/criteria for triggering the alarm. Responsive to the criterion/criteria no longer being met, the minimum length of the silence period may be increased.

As another example, if the Hb (hemoglobin) level of the subject drops this will suggest an increased chance of hypovolemic shock. Accordingly, the minimum length of the silence period may be reduced for any alarm parameters that relate to other hypovolemic shock parameters are reduced.

Preferably, the alarm parameter is derived by processing a plurality of values of physiological parameters.

In other examples, the subject condition information may comprise information that changes responsive to any change in a value that is processed to produce a value for an alarm parameter that triggers the alarm. For instance, if the alarm parameter is an HSI, the subject condition information may indicate any changes in a value of a heartrate.

In some examples, the first information comprises subject intervention information that changes responsive to the occurrence of any clinical interventions performed on the subject. Processing the first information comprises processing at least the subject intervention information. A clinical intervention results from a clinical interaction with the subject.

In particular, the subject intervention information may comprise an indication of the occurrence of any clinical intervention that are predicted/expected to affect or change a value for an alarm parameter (e.g., if the alarm parameter is an HSI, then the clinical intervention may be a hemodynamic intervention such as a fluid injection). The minimum length of the silence period may decrease or increase responsive to one or more such clinical interventions occurring.

For instance, the minimum length of the time period may increase to avoid an alarm being triggered during a known or expected fluctuation of the alarm parameter following intervention. In particular, the minimum length of the silence period may be increased to a length of time that it is expected for the effect of the intervention to take place. It will be appreciated that, after an intervention, the caregiver or clinician is likely to already by monitoring the subject, such that generation of an alarm would be unnecessary.

By way of example, if the subject is provided with vasoconstrictors, then this intervention will impact blood pressure. Accordingly, if the alarm parameter is a blood pressure (or responsive to a change in blood pressure), then the minimum length T_{MIN} of the silence period should be reduced so that a clinician can check that the vasoconstrictors have not caused an adverse effect.

As another example, if the subject undergoes ventilation, then this intervention this will induce a (short) period of increased fluctuations of other hemodynamic parameters. Accordingly, the minimum length T_{MIN} may be increased to allow for this expected time of fluctuation to pass without (re)triggering any the alarms.

In some examples, the first information comprises confidence information that indicates a confidence of a value for an alarm parameter or a value used to derive a value for an alarm parameter. The minimum length of the silence period may decrease responsive to the confidence information indicating that a confidence for such a value has increased.

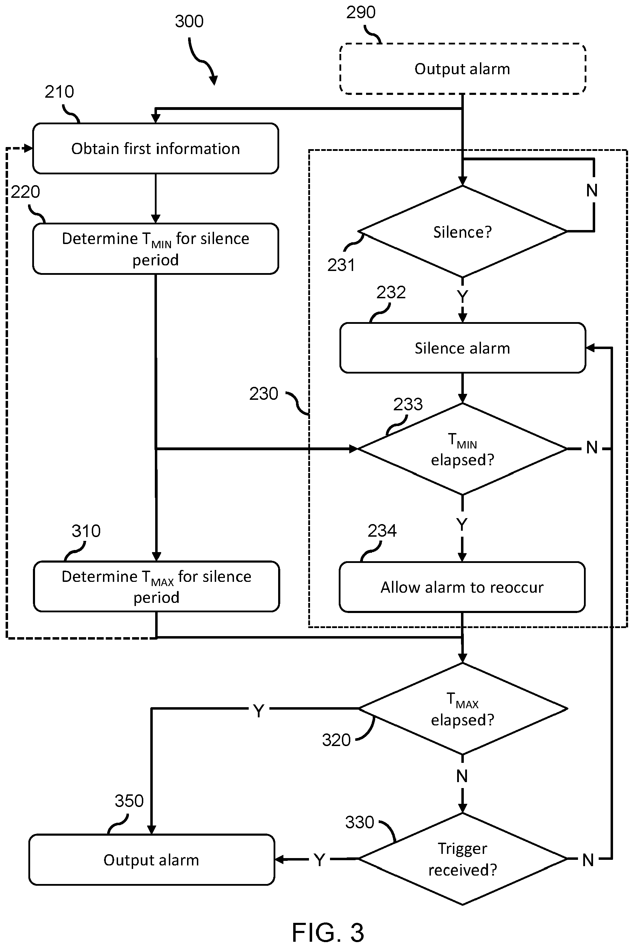

Fig. 3 is a flowchart illustrating a method 300 according to a further embodiment. The method 300 comprises all of the steps of the previously disclosed method 200, which are not repeated for the sake of conciseness. Additional optional steps are hereafter described and illustrated by Fig. 3.

In one example, the method 300 further comprises a step 310 of processing the first information to determine a maximum length for the silence period. Step 310 may be performed in a similar manner to step 220, previously described. In particular, there may be a default value for the maximum length for the silence period which is increased or reduced responsive to information contained in the first information.

In some examples, step 232 further comprises providing a user-perceptible output of the maximum time period. This provides a clinician with useful information (e.g., defining how long until the alarm will sound again) that can influence their clinical decision making. The user perceptible-output may be provided at the same output interface that provides a user-perceptible output of the (original) alarm.

The method 300 also comprises a step 350 of, responsive to receiving an indication to silence the alarm, triggering the reoccurrence of the alarm when the maximum length for the silence period has elapsed. In some examples, step 350 acts as step 290, such that the method 300 is again repeated once the alarm is output.

Thus, the method 300 may comprise a determination step 320 that determines whether or not the time that has elapsed after the alarm has been silenced (e.g., during step 230) has reached the maximum length T_{MAX}. Responsive to a positive determination in step 320, the method moves to step 350 of triggering the reoccurrence of the alarm. Responsive to a negative determination in step 320, the alarm remains silenced (e.g., unless an alarm condition has been reached).

The method 300 may also comprise a determination step 330 of determining whether or not a trigger signal has been received. Responsive to a positive determination in step 330, the method moves to step 350 of triggering the reoccurrence of the alarm. Responsive to a negative determination in step 330, the alarm remains silenced.

A trigger signal may, for instance, be an indication that a clinician is interacting or observing the subject. Approaches for determining whether a clinician is interacting or observing with the subject would be well within the capabilities of a skilled person to carry out, e.g., using image analysis of images captured of the subject or by monitoring for any interactions with a patient monitor for the subject.

As another example, the trigger signal may be a user-provided input signal that acts as an override signal, e.g., provided at an input interface.

As yet another example, the trigger signal may be an indication that the alarm parameter(s) meets an alarm condition. As the minimum length for the silence period has elapsed, the alarm may be triggered at this stage.

One or both of steps 320 and 330 may be omitted in some embodiments.

The skilled person would be readily capable of developing a processing system for carrying out any herein described method. Thus, each step of the flow chart may represent a different action performed by a processing system, and may be performed by a respective module of the processing system.

Embodiments may therefore make use of a processing system. The processing system is configured to control the silencing of one or more alarms, and in particular, to control at least a minimum length for a silence period during which an alarm is silenced. The processing system may therefore be configured to operatively communicate with an output generator that produces one or more user-perceptible outputs for an alarm, e.g., to control the silencing of at least one of these outputs.

The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa. Any reference signs in the claims should not be construed as limiting the scope.

## Claims

1. A computer-implemented method of controlling the reoccurrence of an alarm for a subject undergoing physiological patient monitoring, the computer-implemented method comprising, responsive to the occurrence of an alarm:
obtaining first information that changes responsive to any changes in a condition of the subject and/or any changes in a condition of one or more clinicians responsible for the subject;
processing the first information to determine a minimum length for a silence period, being a period for which the alarm is to remain silent after an indication to silence the alarm is received; and
responsive to receiving the indication to silence the alarm, initiating the silence period and preventing the alarm from reoccurring at least until an elapsed length of the silence period breaches the minimum length for the silence period.

2. The computer-implemented method of claim 1, further comprising, during the silence period, iteratively:
obtaining updated first information; and
processing the updated first information to redetermine the minimum length for the silence period.

3. The computer-implemented method of claim 1 or 2, wherein:
the first information comprises clinician information that changes responsive to an availability of the one or more clinicians to attend to the subject; and
processing the first information comprises processing at least the clinician information.

4. The computer-implemented method of claim 3, wherein the clinician information comprises schedule information that indicates a schedule of the one or more clinicians.

5. The computer-implemented method of claim 3 or 4, wherein the clinician information comprises attention information that indicates a level of attention paid by the clinician to the subject undergoing physiological patient monitoring.

6. The computer-implemented method of any of claims 3 to 5, wherein the clinician information comprises location information that indicates a proximity of the one or more clinicians to the subject undergoing physiological patient monitoring.

7. The computer-implemented method of any of claims 1 to 5, wherein:
the first information comprises subject condition information that changes responsive to any changes in the condition of the subject; and
processing the first information comprises processing at least the subject information.

8. The computer-implemented method of claim 7, wherein:
the alarm is responsive to a value of at least one alarm parameter derived from one or more monitored physiological parameters; and
the subject condition information changes responsive to any changes in the value of each alarm parameter.

9. The computer-implemented method of any of claims 1 to 8, wherein:
the first information comprises subject intervention information that changes responsive to the occurrence of any clinical interventions performed on the subject; and
processing the first information comprises processing at least the subject intervention information.

10. The computer-implemented method of any of claims 1 to 9, further comprising, responsive to receiving an indication to silence the alarm, controlling a user interface to provide a user-perceptible output responsive to minimum length for the silence period.

11. The computer-implemented method of any of claims 1 to 10, further comprising, responsive to the occurrence of an alarm:
processing the first information to determine a maximum length for the silence period; and
responsive to receiving an indication to silence the alarm, triggering the reoccurrence of the alarm when the maximum length for the silence period has elapsed.

12. The computer-implemented method of claim 11, further comprising responsive to receiving an indication to silence the alarm after the occurrence of the alarm, triggering the reoccurrence of the alarm responsive to:
the elapsed length of the silence period falling between the minimum length and the maximum length of the silence period; and
receiving a trigger signal for triggering the reoccurrence of the alarm.

13. The computer-implemented method of claim 12, wherein the trigger signal is an indication that a clinician is interacting or observing the subject.

14. A computer program product comprising computer program code means which, when executed on a computing device having a processing system, cause the processing system to perform all of the steps of the method according to any of claims 1 to 13.

15. A processing system for controlling the reoccurrence of an alarm for a subject undergoing physiological patient monitoring, the processing system being configured to, responsive to the occurrence of an alarm:
obtain first information that changes responsive to a condition of the subject and/or one or more clinicians responsible for the subject;
process the first information to determine a minimum length for a silence period, being a period for which the alarm is to remain silent after an indication to silence the alarm is received; and
responsive to receiving the indication to silence the alarm, initiate the silence period and prevent the alarm from reoccurring at least until an elapsed length of the silence period breaches the minimum length for the silence period.
